(19) Europäisches Patentamt

European Patent Office

Office européen des brevets

(11) Veröffentlichungsnummer: **0 250 802**
**A1**

# (12) EUROPÄISCHE PATENTANMELDUNG

(21) Anmeldenummer: **87106926.6**

(22) Anmeldetag: **13.05.87**

(51) Int. Cl.⁴: **A61K 31/19 , A61K 9/08**

(30) Priorität: **24.06.86 DE 3621036**

(43) Veröffentlichungstag der Anmeldung:
**07.01.88 Patentblatt 88/01**

(84) Benannte Vertragsstaaten:
**AT BE CH DE FR GB IT LI LU NL SE**

(71) Anmelder: **Merckle GmbH**
**Dr.-Georg-Spohn-Strasse 7**
**D-7902 Blaubeuren(DE)**

(72) Erfinder: **Von Stetten, Otto, Dr.**
**Blaubeurerstrasse 91**
**D-7933 Scheiklingen(DE)**
Erfinder: **Schmid, Franz,**
**Mozartstrasse 48**
**D-8068 Pfaffenhofen(DE)**
Erfinder: **Räuchle, Kurt, Dr.**
**Forstweg 16**
**D-7902 Blaubeuren(DE)**
Erfinder: **Seth, Pyare Lal, Dr.**
**Krebsenbachweg 8**
**CH-4147 Aesch/BL(CH)**

(74) Vertreter: **Lederer, Franz, Dr. et al**
**Van der Werth, Lederer & Riederer**
**Patentanwälte Lucile-Grahn-Strasse 22**
**D-8000 München 80(DE)**

(54) **Flüssige Ibuprofen-Zubereitungen.**

㊿ Es werden wässrige, stabile, relativ konzentrierte Lösungen von Ibuprofen, die eine Mischung von Propylenglykol und Polyethylenglykol in definierten Mengenverhältnissen enthalten, offenbart.
Vorzugsweise enthalten die Lösungen ein Lokalanästhetikum wie Lidocain.

EP 0 250 802 A1

### Flüssige Ibuprofen-Zubereitungen

Die Erfindung betrifft stabile flüssige Ibuprofen-Zubereitungen, die besonders für parenterale Wirkstoffapplikation geeignet sind. Ibuprofen ist der international eingeführte Trivialname des Wirkstoffes 2-(4-Isobutylphenyl)propionsäure (IUPAC); es ist ein nicht-steroidales Antirheumatikum, das zur symptomatischen Therapie bei Rheumaerkrankungen eingesetzt wird. Wegen des relativ großen "first-pass-effects" der Substanz und zur raschen Anflutung ist es wünschenswert, Injektionslösungen einzusetzen, wobei eine Dosis von 400-600 mg (bezogen auf die freie Säure) verwendet werden soll. Für intramuskuläre Injektion soll das Volumen jedoch möglichst niedrig gehalten werden.

Wegen der relativen Schwerlöslichkeit von Ibuprofen in Wasser ist eine wässrige Injektionslösung mit vernünftigem Volumen nicht zu erhalten.

Aufgabe der vorliegenden Erfindung war es, injizierbare Ibuprofen-Zubereitungen zur Verfügung zu stellen, die bei kleinem Injektionsvolumen die möglichst schmerzfreie Injektion therapeutisch ausreichender Arzneistoffmengen zulassen und außerdem lagerstabil sind.

Bisher vertriebene Ibuprofen-Injektionslösungen stehen aus Stabilitätsgründen ausschließlich als Lyophilisate des Lysinsalzes zur Verfügung. Zusätze von Lokalanästhetika zur Verminderung hierbei häufig beobachteten Injektionsschmerzes sind nicht enthalten.

Die Konzentration des rekonstituierten bekannten Lyophilisates liegt bei 234,2 mg pro 3 ml fertiger Lösung (bezogen auf die freie Säure). Höhere Konzentrationen sind aufgrund der Löslichkeit des Lysinsalzes nicht möglich.

Bei der Entwicklung einer lagerstabilen Lösung wurden nun die als Lösungsmittel für Parenteralia bekannten Substanzen Propylenglykol und Polyethylenglykol eingesetzt. Der Wirkstoff lag hierbei als Natriumsalz vor. Doch konnten hierbei keine höherkonzentrierten Lösungen erhalten werden.

Es wurde nun überraschend gefunden, daß mit einem Lösungsmittelsystem bestehend aus Wasser und einem Gemisch aus Propylenglykol und Polyethylenglykol in den nachstehend angegebenen Mengenverhältnissen vergleichsweise hochkonzentrierte Wirkstofflösungen zumindest bei höheren pH-Werten erhalten werden können.

Darüberhinaus wurde überraschenderweise festgestellt, daß eine noch bessere Lösung des Ibuprofen erreicht werden kann, wenn dem System zusätzlich ein Lokalanästhetikum, nämlich Lidocain, zugesetzt wird. Hierdurch wird eine ausreichende Lösungsfähigkeit schon bei tieferen, dem physiologischen Wert mehr entsprechenden pH-Werten erreicht.

Die Wirkung des Lidocains ist insbesondere deshalb über raschend, weil zuvor festgestellt wurde, daß das Lösungsmittelsystem bei höherem pH-Wert besser wirkt als bei tieferem pH-Wert und Lidocain-HCl den pH-Wert erniedrigt.

Erfindungsgemäß ist es also möglich, die gewünschte Applikationsmenge von 400-600 mg Ibuprofen (bezogen auf die freie Säure) in nur 3 ml Injektionslösung bei einem pH-Wert von ca. 7,5 in stabiler Lösung zu halten.

Gegenstand der Erfindung sind somit flüssige Ibuprofen-Zubereitungen, insbesondere für die parenterale Applikation, bestehend aus einer Lösung von Ibuprofen oder eines seiner Salze und gegebenenfalls weiteren pharmazeutischen Wirk- und Hilfsstoffen in einem Lösungsmittel aus l0-70 Gew.%, vorzugsweise 20-50 Gew.%, einer Mischung aus a) Propylenglykol und b) Polyethylenglykol und 90-30 Gew.%, vorzugsweise 80-50 Gew.% Wasser, wobei in dem Lösungsmittelgemisch das Gewichtsverhältnis von Propylenglykol : Polyethylenglykol zwischen 9,5 : 0,5 und 0,5 : 9,5 vorzugsweise 3 : l und l : 3, besonders bevorzugt zwischen 2 : l und l : 2 liegt.

Im allgemeinen enthalten die Zubereitungen 5-27 Gew.% Ibuprofen (freie Säure), vorzugsweise l2-20 Gew.% Ibuprofen.

Als Polyethylenglykol wird vorzugsweise Polyethylenglykol 400 eingesetzt.

Die Konzentration eines zugesetzten Lokalanästhetikums, insbesondere Lidocains, kann zwischen 0,l Gew.% und 5 Gew.% schwanken und liegt bevorzugt im Bereich von 0,5 Gew.% bis 2 Gew.%, bezogen auf Lidocain-HCl. Der Zusatz eines Lokalanästhetikums ist auch aus medizinischen Gründen zur Verhinderung des Injektionsschmerzes bei intramuskulärer Injektion nicht unerwünscht.

Neben der üblichen intramuskulären Injektion von Antirheumatika ist es auch möglich die erfindungsgemäßen Zubereitungen intravenös zu applizieren. Bei der Applikation am Tier (Beagle Hunde) konnten hierbei keine unerwünschten Wirkungen beobachtet werden.

Bei den erfindungsgemäßen Zubereitungen ist es möglich, den pH-Wert im Bereich zwischen pH = 6,5 und pH = 8 einzustellen.

In der Therapie der rheumatischen Erkrankungen ist es häufig angezeigt, zur Wirkungsverstärkung neben Ibuprofen weitere antiinflammatorische Stoffe, vorzugsweise Steroide, z.B. Glucocorticoide wie Dexamethason oder Prednisolon, zu verabreichen. In üblicherweise verwandten Lösungsmitteln sind Steroide oder geeignete Derivate neben Ibuprofen relativ schlecht löslich.

Bei den erfindungsgemäßen Zubereitungen ist es jedoch möglich in für i.m. Injektion bevorzugten Volumina, z.B. 3 ml, neben 400-600 mg Ibuprofen auch größere Mengen Steroid, z.B. 25 mg Prednisolon in Form von 33 mg Prednisolon-2l-phosphat-dinatriumsalz, lagerstabil zu lösen.

Alle Prozentangaben beziehen sich auf die komplette Injektionslösung.

Beispiel l:

400 g Ibuprofen werden in einer Lösung aus 720 g Propylenglykol, 540 g Polyethylenglykol 400, 30 g Lidocain-HCl und 900 g Wasser suspendiert. Anschließend wird l0%ige Natronlauge zur Lösung des Ibuprofens zugesetzt und auf pH 7,5 eingestellt.

Anschließend wird mit Wasser bis zu einem Volumen von 3 Litern aufgefüllt.

Die Lösung wird sterilfiltriert und in Ampullen zu 3 ml abgefüllt und bei l20°C 20 Minuten sterilisiert.

Die in Ampullen abgefüllte Lösung wurde l Jahr bei Raumtemperatur gelagert und ist stabil.

Beispiel 2:

600 g Ibuprofen werden in einer Lösung aus 720 g Propylenglykol, 540 g Polyethylenglykol 400, 30 g Lidocain-HCl und 800 g Wasser suspendiert. Anschließend wird l0%ige Natronlauge zur Lösung des Ibuprofens zugesetzt und auf pH 7,5 eingestellt.

Anschließend wird mit Wasser bis zu einem Volumen von 3 Litern aufgefüllt.

Die Lösung wird sterilfiltriert und in Ampullen zu 3 ml abgefüllt und bei l20°C 20 Minuten sterilisiert.

Beispiel 3:

Es wird eine Lösung nach Beispiel l hergestellt, jedoch werden zusätzlich 33 g Prednisolon-2l-phosphat-dinatrium zugesetzt. Die Lösung wird sterilfiltriert und in Ampullen zu 3 ml abgefüllt und bei l20°C 20 Minuten sterilisiert.

**Ansprüche**

l. Flüssige Ibuprofen-Zubereitungen, insbesondere für die parenterale Applikation, speziell zur intramuskulären und intravenösen Anwendung, bestehend aus einer Lösung von Ibuprofen oder eines seiner Salze und gegebenenfalls weiteren pharmazeutischen Wirkstoffen und Hilfsstoffen in einem Lösungsmittel, dadurch gekennzeichnet, daß das Lösungsmittel aus l0-70 Gew.%, vorzugsweise 20-50 Gew.%, einer Mischung aus a) Propylenglykol und b) Polyethylenglykol und 90-30 Gew.%, vorzugsweise 80-50 Gew.% Wasser besteht, wobei in dem Lösungsmittelgemisch das Gewichtsverhältnis von a) Propylenglykol zu b) Polyethylenglykol zwischen 9,5 : 0,5 und 0,5 : 9,5, vorzugsweise zwischen 3 : l und l : 3 liegt.

2. Ibuprofen-Zubereitungen nach Anspruch l, dadurch gekennzeichnet, daß in dem Lösungsmittelgemisch das Gewichtsverhältnis von a) Propylenglykol zu b) Polyethylenglykol zwischen 2 : l und l : 2 liegt.

3. Ibuprofen-Zubereitungen nach einem der vorgehenden Ansprüche, dadurch gekennzeichnet, daß sie einen pH-Wert von 6-8,5, insbesondere 6,5-8,0 aufweisen.

4. Ibuprofen-Zubereitungen nach einem der vorgehenden Ansprüche, dadurch gekennzeichnet, daß sie als Polyethylenglykolkomponente ein Polyethylenglykol 400 enthalten.

5. Ibuprofen-Zubereitungen nach einem der vorgehenden Ansprüche, daduch gekennzeichnet, daß sie außerdem 0,l-5 Gew.%, vorzugsweise 0,5-2 Gew.%, eines Lokalanästhetikums enthalten.

6. Ibuprofen-Zubereitungen nach Anspruch 5, dadurch gekennzeichnet, daß das Lokalanästhetikum Lidocain ist.

7. Ibuprofen-Zubereitungen nach Anspruch 6, dadurch gekennzeichnet, daß sie einen pH-Wert von 7-8 aufweisen.

8. Ibuprofen-Zubereitungen nach einem der vorgehenden Ansprüche, dadurch gekennzeichnet, daß sie 5-27 Gew.% Ibuprofen enthalten.

9. Ibuprofen-Zubereitungen nach einem der vorgehenden Ansprüche, dadurch gekennzeichnet, daß sie zusätzlich einen antiinflammatorisch wirksamen Arzneistoff, vorzugsweise ein Steroid, enthalten.

l0. Ibuprofen-Zubereitungen nach Anspruch 7, dadurch gekennzeichnet, daß sie im wesentlichen aus l2-20 Gew.% Ibuprofen als Natriumsalz, etwa 24 Gew.% Propylenglykol, etwa l8 Gew.% Polyethylenglykol 400, etwa l Gew.% Lidocain-Hydrochlorid, Rest Wasser besteht, wobei die Lösung mit Natronlauge auf einen pH-Wert von etwa 7,5 eingestellt ist.

Europäisches
Patentamt

**EUROPÄISCHER RECHERCHENBERICHT**

Nummer der Anmeldung

| | EINSCHLÄGIGE DOKUMENTE | | EP 87106926.6 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE - A1 - 2 508 895 (SPA SOCIETA PRODOTTI ANTIBIOTICI S.P.A.)<br><br>* Ansprüche 1-4,8,9,14,18; Seite 16, letzter Absatz *<br><br>-- | 1 | A 61 K 31/19<br>A 61 K 9/08 |
| Y | EP - A2 - 0 137 668 (THE UPJOHN COMPANY)<br><br>* Anspruch 1; Seite 3, Zeilen 23-25; Seite 4, Zeilen 4-8 *<br><br>-- | 1 | |
| A | US - A - 4 282 252 (A.M. LEFER)<br><br>* Zusammenfassung; Ansprüche 1,7 *<br><br>-- | 1 | |
| A | DE - A1 - 3 340 347 (THE UPJOHN COMPANY)<br><br>* Zusammenfassung; Beispiele 11,12 *<br><br>-- | 1,3,4, 7 | **RECHERCHIERTE SACHGEBIETE (Int. Cl.4)** |
| A | EP - A1 - 0 070 714 (THE UPJOHN COMPANY)<br><br>* Anspruch 4; Beispiel 5 *<br><br>-- | 1,3,7 | A 61 K 31/00<br>A 61 K 9/00 |
| A | EP - A1 - 0 068 838 (THE UPJOHN COMPANY)<br><br>* Ansprüche 2,3; Beispiel 5 *<br><br>-- | 1,3,5, 7 | |
| A | AT - B - 369 651 (A. NATTERMANN & CIE)<br><br>* Anspruch 1 * | 1,3,7 | |

Der vorliegende Recherchenbericht wurde für alle Patentansprüche erstellt.

| Recherchenort | Abschlußdatum der Recherche | Prüfer |
|---|---|---|
| WIEN | 01-10-1987 | MAZZUCCO |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | **EINSCHLÄGIGE DOKUMENTE** | | EP 87106926.6 |
| A | EP - A1 - 0 178 436 (DOLORGIET GMBH & CO.KG)<br><br>* Zusammenfassung; Ansprüche 1,2; Beispiele 1-3,5,12,13, 16 * | 1,3,4, 7,8 | |
| A | US - A - 4 393 076 (K. NODA et al.)<br><br>* Zusammenfassung; Ansprüche 1,4,11 * | 1,3,7, 8 | |
| A | FR - A - 1 320 713 (J.R. GEIGY)<br><br>* Ansprüche 1,2 * | 1,3,5, 7 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 01-10-1987 | MAZZUCCO |